# EUROPEAN PATENT APPLICATION

(11) **EP 1 074 265 A1**
(43) Date of publication of application: **07.02.2001**
(21) Application number: 99202549.4
(22) Date of filing: 03.08.1999
(51) Int. Cl.: A61K 38/22, G01N 33/74, A61P 15/08, A61P 15/18

(54) **Use of AMH and/or AMH agonists and/or AMH antagonists for long-term control of female fertility**

(71) Applicant: Erasmus Universiteit Rotterdam, 3015 GE Rotterdam (NL)
(72) Inventor: Grootegoed, Johan Anton, 2991 KS Barendrecht (NL); Themmen, Axel Peter Nico, 3311 DL Dordrecht (NL)
(74) Representative: Ottevangers, Sietse Ulbe

(57) **Abstract**

Method of treatment of a woman to modify her fertility comprising administering anti-Müllerian hormone (AMH) and/or an AMH agonist and/or an AMH antagonist. Said treatment may be used to inhibit recruitment of primordial follicles into the pool of growing follicles. Contraception, prolongation of the duration of fertility and postponement of the menopause may result therefrom. Said treatment may also be directed to promote recruitment of primordial follicles into the pool of growing follicles. Improved fertility may result therefrom.

## Description

### Background of the invention

Anti-Müllerian hormone (AMH), also called Müllerian inhibiting substance (MIS) or Müllerian inhibiting factor (MIF), is a member of the transforming growth factor β (TGFβ) superfamily of peptide growth and differentiation factors. During male fetal sex differentiation, AMH is synthesized by the testicular Sertoli cells and induces degeneration of the Müllerian ducts, which form the anlagen of the uterus, the oviducts and the upper part of the vagina. During female fetal development, no ovarian AMH production occurs. However, AMH mRNA expression is detected in ovarian granulosa cells from postnatal day 3 onwards. Immunohistochemical and mRNA in situ hybridisation studies in rats revealed specific expression of AMH and its type II receptor (AMHRII) in granulosa cells of mainly non-atretic pre-antral and small antral follicles, while the signal is lost in non-atretic large antral follicles and atretic follicles of all size classes. During the estrous cycle, no marked changes are detected in the patterns of AMH and AMHRII mRNA expression, except at estrus, when a heterogeneous decrease in expression of both mRNA's is found in non-atretic pre-antral follicles compared to the more homogeneous expression pattern on other days of the cycle. In cultured rat granulosa cells, exogenous AMH inhibits biosynthesis of aromatase and decreases LH receptor number. Furthermore, AMH opposes proliferation of cultured granulosa-luteal cells.

In females, AMH is produced exclusively in the ovary by growing follicles. The marked changes in AMH and AMHRII mRNA expression and the reported effects of AMH in in vitro culture systems indicate that AMH may play a role during follicle development in the postnatal ovary. However, little is known about the exact function of AMH in the ovary.

Behringer et al. 1994, Cell 79: 415-425, "Müllerian-inhibiting substance function during mammalian sexual development" studied the function of AMH with the help of an AMH null (AMH⁻) mouse model. AMH null males develop Müllerian duct derivatives, including oviducts, a uterus, and a vagina, in addition to a complete male reproductive system. AMH null females have macroscopically normal uteri, oviducts and ovaries. Furthermore, these females are fertile and have litters of normal size.

Based on the above (Behringer et al. 1994) and other (Mishina et al. 1996, Genes Dev. 10: 2577-2587; Mishina et al. 1999, Endocrinology 140: 2084-2088) mouse knockout models [AMH(-/-) and/or AMHRII(-/-) mice], it has been concluded that AMH does not play a prominent role in control of follicle development or other aspects of ovarian function.

### Summary of the invention

Notwithstanding the above mentioned perception in the art, we studied the function of AMH in the postnatal ovary with the help of the above mentioned AMH null mouse model, starting from the hypothesis that AMH might be involved in more subtle and/or long-term aspects of control of follicle development and/or follicle selection. In particular, to investigate whether AMH exerts effects on the composition of the follicle population, we determined the entire follicle population in wild type mice [AMH (+/+)], mice heterogeneous for the AMH null mutation [AMH (+/-)] and AMH null mice [AMH (-/-)] at 25 days, 4 months and 13 months of age. Furthermore we also determined other parameters of reproductive function, such as serum FSH and inhibin levels and ovarian and uterine weights.

Surprisingly, our research revealed that AMH is an important regulator of primordial follicle recruitment and that ovaries of AMH null mice show depletion of primordial follicles at an earlier age. Apparently, the absence of AMH in AMH gene knockout mice results in increased recruitment of primordial follicles into the pool of growing follicles.

Based on this surprising observation, we conceived that exogenous AMH and/or AMH agonists and/or AMH antagonists can be used to control the recruitment of primordial follicles and the size of the primordial follicle pool in women.

According to the present invention, women of various ages can be treated with AMH and/or AMH agonists and/or AMH antagonists for various reasons, such as contraception, extension of the fertile period, postponement of menopause, control of follicle development, etc.

Furthermore, the present invention allows to determine the circulating level of AMH in blood of women of various ages to obtain a parameter to predict/estimate the length of the fertile period and/or to evaluate the course of the menopause.

### Brief description of the drawings

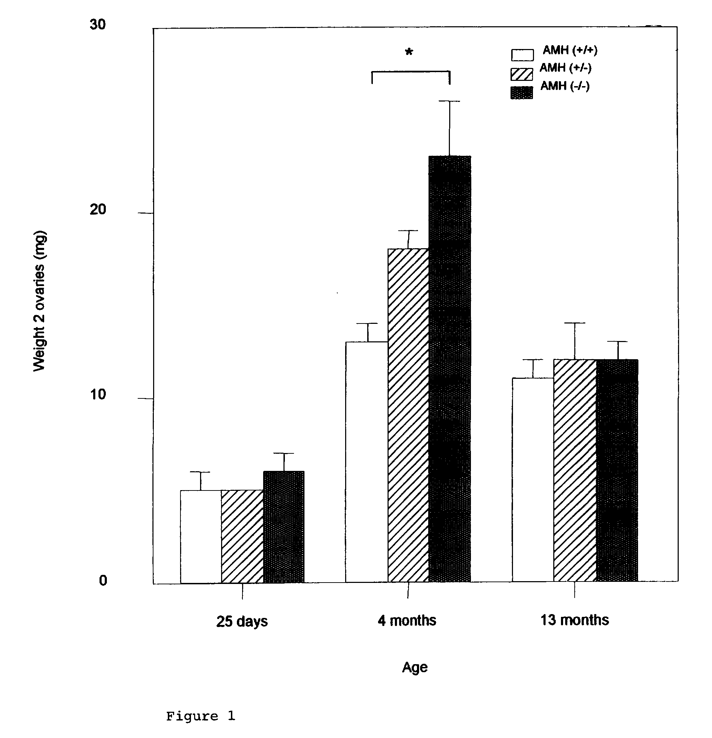

Figure 1 is a graph showing the ovarian weight in 25-day, 4-month and 13-month old AMH (-/-), AMH (+/-) and AMH (+/+) female mice. The combined weight of both ovaries is given. At 4 months of age, ovarian weight was significantly higher in AMH (-/-) females than in AMH (+/+) females. Data represent the mean ± SEM (N = 4-5). The asterisk indicates a statistically significant difference (P ≤ 0.05).

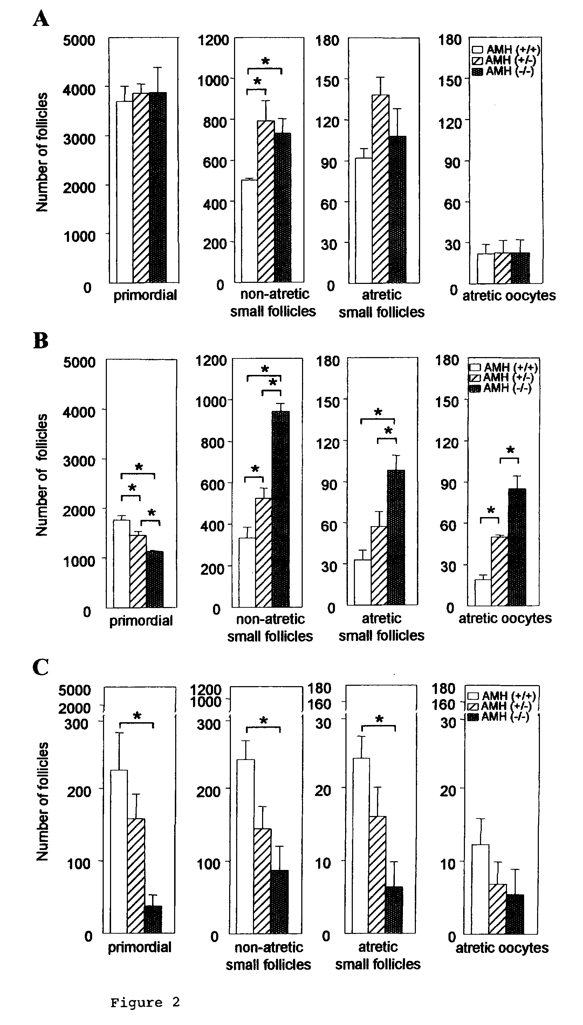

Figure 2 is a graph showing the follicle population in 25-day, 4-months and 13-months old AMH (+/+), AMH (+/-) and AMH (-/-) females. Data represent the mean ± SEM (N = 4-5). The asterisks indicate a statistically significant difference (P ≤ 0.05).

A: Follicle population in 25-day old AMH (-/-), AMH (+/-) and AMH (+/+) female mice. Significant more non-atretic small follicles were detected in AMH (+/-) females and AMH (-/-) females than in AMH (+/+) females. No significant difference in the number of primordial follicles, atretic small follicles and atretic oocytes was observed between the three groups of mice.

B: Follicle population in 4-month old AMH (-/-), AMH (+/-) and AMH (+/+) female mice. Significant less primordial follicles and significantly more non-atretic small follicles were found in AMH (-/-) and AMH (+/-) females than in AMH (+/+) females. Significantly more atretic small follicles were found in AMH (-/-) females than in the two other groups of females, while significantly more atretic oocytes were found in AMH (-/-) and AMH (+/-) females than in AMH (+/+) females.

C: Follicle population in 13-month old AMH (-/-), AMH (+/-) and AMH (+/+) female mice. Significant less primordial follicles and non-atretic and atretic small follicles were found in AMH (-/-) females than in AMH (+/+) females.

### Detailed description of the invention

This invention provides a method of treatment of a woman to modify her fertility comprising administering anti-Müllerian hormone (AMH) and/or an AMH agonist and/or an AMH antagonist to said woman in an amount effective to modify fertility.

The method may seek to inhibit recruitment of primordial follicles into the pool of growing follicles, in particular in order to obtain contraception and/or prolong the duration of fertility and/or postpone the menopause. It is preferred to administer AMH and/or an AMH agonist for this purpose and preferably it is administered in combination with a different contraceptive. (The phrase 'in combination with' as used in the application includes all forms of combined use, including simultaneous or subsequent administration, by the same or a different medicament, by the same or a different route of administration.)

Alternatively, the method may rather aim at promoting recruitment of primordial follicles into the pool of growing follicles, for example to treat ovarian failure caused by lack of proper recruitment of primordial follicles into the pool of growing follicles and/or to promote fertility and/or to shorten the duration of the menopause. For these goals, it is preferred to administer an AMH antagonist and preferably, when improved fertility is aimed at, in combination with a fertility hormone, like FSH or LH.

The invention provides anti-Müllerian hormone (AMH), an AMH agonist or an AMH antagonist for use in human medical therapy, in particular for modifying (such as preventing or delaying), or rather the opposite, i.e. promoting, fertility in women.

This invention relates to the use of anti-Müllerian hormone (AMH) and/or an AMH agonist and/or an AMH antagonist in the manufacture of a medicament for modifying fertility in women.

The invention furthermore provides a pharmaceutical formulation comprising anti-Müllerian hormone (AMH) and/or an AMH agonist and/or an AMH antagonist in combination with a physiologically acceptable diluent or carrier. Such pharmaceutical formulation may particularly be intended for modifying fertility in women.

The invention also provides a method of estimating the remaining duration of the fertile period and/or the onset of the menopause and/or the course of the perimenopausal transition of a woman comprising performing an assay on an appropriate tissue or body fluid sample from said woman, for example a blood, plasma or serum sample, to determine the anti-Müllerian hormone (AMH) level.

AMH is produced exclusively by the ovary and acts exclusively on the ovary. The highly specific AMH type II receptor is present exclusively in the ovary. Hence, exogenous AMH and/or AMH agonists and/or AMH antagonists will act exclusively on the ovary. AMH production and action is confined to primordial follicles (action) and primary and small secondary follicles (production and action).

First, the invention implies that AMH, AMH agonists and/or AMH antagonists can be used for contraception, by inhibition of recruitment of primordial follicles into the pool of growing follicles. Current hormonal methods for contraception do not inhibit this recruitment. A major advantage of the invention is, that contraception based on exogenous AMH and/or AMH agonists and/or AMH antagonists will be associated with conservation of the pool of primordial follicles. Conservation of this pool is essential to obtain optimal conditions for a prolonged period of fertility following the end of the contraceptive period.

Second, the present invention implies that AMH and/or AMH agonists and/or AMH antagonists can be used to prolong the fertile period and postpone the menopause. Menopause is a major milestone in the ageing process in women. The cessation of ovarian function and the almost complete absence of female sex steroid hormone production by the ovaries in postmenopausal life contributes to increased manifestation of osteoporosis, cardiovascular disease and Alzheimer's disease. Menopause is caused by the exhaustion of the pools of ovarian follicles. The number of primordial follicles, which are the source of all growing follicles in the cycling ovary, is determined at birth and declines from that time to approx. 38 years of age in a logarithmic pattern. Depletion of the follicle pools leads to menopause.

Third, the subject invention implies that new methods for treatment of different types of ovarian failure will become available. Current hormonal treatments for the control of follicle growth and development are directed towards the secondary and larger follicles. Pharmaceutical agents which influence the development of primordial and primary follicles are not available. AMH and/or AMH agonists and/or AMH antagonists can be used for this purpose.

Cells transfected with DNA (preferably a cDNA construct, but genomic DNA or synthetic DNA are useful as well) encoding human AMH can be used for production of recombinant AMH. DNA encoding human AMH is known from the literature (Cate et al. 1986, Cell 45: 685-698; "Isolation of the bovine and human genes for Mullerian inhibiting substance and expression of the human gene in animal cells"). The DNA encoding human AMH can be placed in a suitable expression vector containing a strong enhancer/promoter sequence that controls AMH mRNA expression. Suitable vectors, expression regulatory elements, etc., are well known to the person skilled in the art. Upon stable transfection in a suitable cell line, such as HEK 293 cells, in combination with a sequence which encodes furin, an enzyme that will cleave the AMH precursor protein at the suitable site, AMH can be isolated from the supernatant of the cultured cells by using suitable antibodies [Nachtigal & Ingraham 1996, Proc. Natl. Acad. Sci. USA 93: 7711-7716; Wilson et al. 1993, Mol. Endocrinol. 7: 247-257]. Other host cells suitable for recombinant production of a heterologous protein such as AMH (in particular eukaryotic, especially mammalian cells, in view of the fact that AMH is a glycoprotein) are well known to the person skilled in the art. This also applies to other proteolytic enzymes than furin which are useful for the proper processing of hormone precursors. It is also possible to first produce the recombinant AMH precursor and then, after its isolation from the cells and/or cell medium, treat it under proper conditions with a suitable processing enzyme in order to obtain the functional mature AMH. Alternatively, the mature AMH may be expressed directly without involvement of a precursor stage.

Of course, instead of producing and using recombinant AMH, it is also possible to use AMH isolated from a natural source of it. Practically however, use of recombinant AMH will be preferred.

The phrase 'AMH, AMH agonists and AMH antagonists' as used herein intends to cover any fragment or derivative of AMH showing the function of regulating primordial follicle recruitment or antagonising said function of AMH.

Base sequence alterations, substitutions, insertions or deletions of the DNA encoding the human AMH can be used to produce different mutant human AMH proteins. These mutant AMH proteins may cause AMH to be more active through:
- increased stability in the circulation
- binding with higher affinity to the AMH receptor complex
- activating the receptor complex more efficiently
- a decreased induction of receptor internalization or down-regulation.

The AMH mutations can be localized in the pro-region of the protein and predominantly stimulate optimal protein folding, stimulate optimal protein cleavage to release the mature peptide, or stimulate optimal protection of the pro-region to the mature peptide during secretion and transport. The mutations can be localized in the cleavage site between the pro-region and the mature peptide in the AMH precursor protein, to stimulate optimal cleavage to release the mature peptide. The mutations can be localized in the mature peptide part of the AMH protein, to increase stability of the peptide, increase receptor binding, increase AMH efficacy on the receptor activity, decrease receptor internalization and/or down-regulation.

In addition, mutant AMH proteins can be produced which act as AMH antagonists. Such mutations can be placed in the AMH precursor protein, to produce protein having AMH antagonistic activity. The mutations can be localized in the pro-region of the AMH precursor protein, to inhibit optimal protein cleavage to release the mature peptide. The mutations can be localized in the cleavage site itself, resulting in an uncleaved AMH antagonist that blocks the activity of AMH [Mishina et al. 1999]. The mutations may also be localized in the mature peptide region of the AMH precursor protein, precluding receptor activation without limiting binding of the antagonist to the AMH receptor complex.

The activity of AMH agonists and antagonists can be tested using the following systems:
- culture of urogenital ducts isolated from embryos or fetuses from pregnant wild type and AMH knockout strains. These ducts can be incubated with various AMH preparations and the regression of the Müllerian ducts can be assessed [MacLaughlin et al. 1992, Endocrinology 131: 291-296].
- incubation of HEK293 cells stably transfected with cDNAs encoding AMH type II receptor cDNA, AMH type I receptor (Alk2), a reporter construct containing the aromatase promoter linked to luciferase cDNA, FSH receptor cDNA (all cDNAs can be taken from different species, including rodents and human). The inhibitory effect of AMH on the FSH-induced aromatase-luciferase response is the parameter to be determined in this system (our observations). This system can be used to test the various AMH preparations.
- other cell culture reporter systems.

Since AMH is an important determinant of the number of primordial follicles recruited into the growing pool, and therefore also in the determination of the number of small growing follicles, serum levels of AMH can be used as a parameter to predict the remaining duration of the fertile period and the course of the perimenopausal transition. When the primordial follicle pool has become smaller, less growing follicles will be formed and serum AMH levels will become lower. Thus, for women close to the menopausal transition, i.e. women approaching 45-50 years of age, a more exact prediction of the age of menopause allows a more precise administration of hormone replacement therapies, and is therefore of great clinical value.

In addition, also in women with a mild form of premature ovarian failure resulting in a more rapid decrease in the primordial follicle pool, serum AMH levels will decline. For these women, a prediction of the length of the remaining fertile period is of great importance, especially if they wish to postpone having children.

AMH and its agonists/antagonists may be administered through various formulations and methods which can be determined easily by persons skilled in the art. In particular, various parenteral administration routes and formulations would be suitable, including in particular administration by intravenous injection or infusion. However, the intramuscular and subcutaneous administration routes can be used as well. Preferable administration routes would include:
- injection of a slowly releasing depot preparation, allowing longer time intervals between injections
- administration as a nose-spray preparation, allowing shorter time intervals between administrations.

Suitable diluents or carriers, which are physiologically acceptable, are well known to persons skilled in the art. For injection or infusion purposes, physiological saline or other suitably buffered aqueous media can be mentioned as useful diluents/carriers.

The optimal dosis to be given and the optimal dosage regimen can be easily determined by persons skilled in the art. Dosis and dosage regimen may be varied according to various parameters, such as the effect sought and the age, weight and condition of the woman to be treated. Also the activity of the selected AMH agonist or antagonist is to be taken into consideration when determining the dosis and dosage regimen.

AMH and its agonists/antagonists can be administered to female patients or healthy females of various ages, during time periods ranging from days to years.

For contraception, AMH and/or its agonists will inhibit the recruitment of primordial follicles from the ovarian pool. AMH and/or its agonists may be administered in combination with existing or novel methods for hormonal or non-hormonal contraception, to obtain contraception with the additional beneficial effect of saving the primordial follicle pool, and thus extending the length of the fertile period following the end of the contraceptive period.

For pro-fertility treatment not associated with the aim of contraception, in woman with aberrant follicular development and/or in woman who wish to postpone the onset of menopause, AMH and/or its agonists/antagonists will control the recruitment of primordial follicles. Treatment with AMH and/or its agonists/antagonists can be combined with treatment with other hormones, including the gonadotropins follicle-stimulating hormone FSH and/or luteinizing hormone LH (recombinant or purified gonadotropin preparations).

The invention will now be illustrated further by the experimental section below, which only serves to illustrate the invention and does not intend to restrict it.

### Materials and Methods

### Animals

A single male and a single female AMH (+/-) mouse were obtained from the Jackson Laboratory, Bar Harbor, Maine, USA. This pair was used to generate F1 mice. Several F1 AMH (-/-) males and females were then crossbred with female and male C57B6 mice to obtain AMH (+/-) mice on a C57B6 genetic background. Subsequently, these mice were used to make AMH (+/+), AMH (+/-) and AMH (-/-) mice, which were used for follicle population analysis. The mice were kept under standard animal housing conditions in accordance with the NIH Guidelines for the Care and Use of Experimental Animals. Lights were on from 7.30 am to 8.30 pm. AMH (+/+) mice, AMH (+/-) mice and AMH (-/-) mice of 25 days (n = 4), 4 months (n = 4) and 13 months of age (n = 5) were used for this study. The 4- and 13-month-old animals were killed on the day of estrus, which was determined by placing the females individually with an AMH (+/+) male of proven fertility in the afternoon, followed by a check for a copulatory plug the next morning. Females with a copulatory plug were killed on the same day at 4 pm by decapitation, after which blood was collected immediately. Females of 25 days old, which are still prepubertal, were also killed at 4 pm. After bleeding, the ovaries were removed, weighed and fixed overnight in Bouin's fluid for histological examination of the follicle population. The uterus was also removed and weighed.

The fixed ovaries were embedded in paraffin after routine histological procedures and 8 µm sections were mounted on slides and stained with hematoxylin and eosin. Blood samples were stored overnight at 4°C and centrifuged the following day at 3000 rpm for 15 min at 4°C. Serum samples were stored at -20°C until assayed for FSH and inhibin.

### Determination of mouse AMH genotype

To analyze the genotype of the mice, genomic DNA was isolated by incubating tail tissue overnight at 55°C in 500 µl 50 mM Tris-HCl (pH 8.0), 100 mM EDTA (pH 8.0), 900 µg/ml proteinase K (Boehringer Mannheim, Mannheim, Germany) and 0.5% (w/v) SDS. The next day, 500 µl phenol (Merck, Darmstadt, Germany) dissolved in Tris-HCl (pH 8.0) was added, and the mixture was shaken vigorously for 15 sec. Subsequently, the solution was centrifuged at 13000 rpm for 10 min. To the supernatant 200 µl phenol and 200 µl chloroform-isoamylalcohol (24:1) were added. After shaking vigorously the solution was centrifuged for 10 min at 13000 rpm. To the supernatant 2 volumes 100% ethanol were added, and the precipitated DNA was washed with 70% ethanol. After the DNA was air-dried for a few minutes, it was dissolved in 100 µl TE buffer (10 mM Tris-HCl, pH 7.5, and 1 mM EDTA, pH 8.0). Approximately 1 µg DNA was used in the PCR reactions.

Primers KO568 (5'-GGAACACAAGCAGAGCTTCC-3') and KO810 (5'-GAGACAGAGTCCATCACG-ACC-3') were used to determine the presence of the wild type allele and were suggested by the Jackson Laboratory. Primer KO568 anneals to nucleotide sequence 568-587 located in exon 1 of the amh gene (numbering according to Genbank sequence: accession number X63240), while primer KO810 anneals to antisense sequence 810-789 located in intron 1, resulting in a PCR product of 243 basepairs. In animals containing the AMH null allele, part of exon 1, intron 1 and exon 2 are replaced by the pCMl neo-cassette. Therefore, the PCR product is only produced in DNA of AMH (+/+) and AMH (+/-) animals which contain the complete wild type allele.

Primers p126 (5'-CTCGTCAAGAAGGCGATA-3') and p127 (5'-GGGATCGGCAT-TGAACA-3') were used to determine the presence or absence of the AMH null allele, i.e. the pCM1 neo-cassette (Thomas & Capecchi 1987, Cell 51: 503-512). Primer p126 anneals to the antisense sequence 1057-1041, while primer p127 anneals to nucleotide sequence 265-281 in the pCMl neo-cassette, resulting in a PCR product of 793 basepairs. Only AMH (+/-) and AMH (-/-) animals which contain the pCMl neo-cassette will produce this PCR product. For the PCR reaction, 50 ng of primers KO568 and KO810, or 100 ng of primers p126 and p127 were used in a PCR buffer containing 1.2 mM dithiothreitol, 10 mM Tris-HCl (pH 8.3), 50 mM KCl, 1.5 mM MgCl₂, 0.25 mM deoxy-NTPs (Pharmacia, Uppsala, Sweden), 0.5 mM spermidine (Sigma, St. Louis, MO, USA) and 0.2 U Supertaq (Sphearo Q, Leiden, The Netherlands) in a total volume of 25 µl.

The PCR reaction mixtures were preheated at 94°C for 5 min followed by 30 PCR cycles (1 min denaturation at 94°C, 1 min annealing at 58°C for the wild type allele and 1 min annealing at 62°C for the null allele, 1 min extension at 72°C) and a final extension step of 10 min at 72°C. The DNA control PCR was carried out as previously described by Slegtenhorst et al. 1998 (Endocrinology 139: 156-162). The products were electrophoresed on a 1.5% agarose gel.

### Estrous cycle length

Daily vaginal smears were taken of female mice belonging to the three different genotypes for a continuous period of 40 days, beginning at three to four months of age (n = 10 per group), to assess cycle length and the regularity of the cycle. Dried smears were examined microscopically and the stage of the estrous cycle was determined according to the criteria of Allen 1922, Am. J. Anat. 30: 297-371. The females were housed individually in cages placed next to a cage containing an adult AMH (+/+) male.

### Ovarian histology and follicle counting

Serial 8 µm sections of both ovaries were used for follicle counting. All primordial follicles were counted in every second section. Based on the mean diameter of the follicle, which was determined by measuring two perpendicular diameters in the section in which the nucleolus of the oocyte was present, the growing follicles were divided into two classes, i.e. small and large follicles. The small follicle class contains preantral and small antral follicles with a diameter smaller than 310 µm and the large follicle class contains antral follicles with a diameter larger than 310 µm. The number of large follicles was very small in females of all three ages. This was expected, since in immature 25-day-old animals growing follicles become atretic before they reach this stage of follicular development and the cycling animals of 4 and 13 months of age were killed on the day of estrus, a time point during the estrous cycle when no large follicles have developed yet.

Non-atretic and atretic follicles were counted separately. The criteria for atresia were the presence of pyknotic nuclei in the granulosa cells and/or degeneration of the nucleus of the oocyte. For all three genotypes we found degenerating, i.e. fragmented oocytes, which are the remnants of atretic follicles. No diameter could be determined of these follicle remnants because the layer of granulosa cells could not very well be distinguished from the surrounding interstitial tissue. To prevent double counting of these follicle remnants, which we called atretic oocytes, these oocytes were counted in every tenth section. The atretic oocytes were classified as a separate group.

In addition, the total number of fresh corpora lutea was counted in the 4- and 13-month-old animals. Newly formed corpora lutea could be distinguished from the older ones by their smaller size of luteal cells.

### Measurements of serum FSH and inhibin

Serum FSH was determined by radioimmunoassay using rat FSH as a ligand and antibodies against ovine FSH (Dullaart et al. 1975, J. Reprod. Fertil. 43: 189-192). All results are expressed in terms of NIADDK-rat FSH-RP-2. The intra-assay variation was 8.8%, and all samples were measured in one assay.

Serum inhibin-like immunoreactivity was estimated using the method described by Robertson et al. 1988 (Mol. Cell. Endocrinol. 58: 1-8), with a bovine follicular fluid preparation with arbitrary potency of 1 U/µg protein as standard. The antibody was raised against purified 32 kDa bovine follicular fluid inhibin and cross-reacts with free α subunits. All samples were run in the same assay and the intra-assay coefficient of variation was 8.2%.

### Statistical analysis

Results are presented as the mean ± SEM. The data were evaluated for statistical differences by 1-way analysis of variance (ANOVA), followed by a Duncan's new multiple range test using SPSS 7.5 (SPSS Inc., Chicago, IL, USA) computer software. Differences were considered significant at P ≤ 0.05.

### Results

### Estrous cycle length

Vaginal smears were taken daily for a period of 40 days to determine the length and regularity of the estrous cycle in AMH (+/+), AMH (+/-) and AMH (-/-) female mice. No differences were found between these three groups of mice, in either the length or the regularity of the estrous cycle. In all three groups some animals showed at least one prolonged estrous cycle (varying from 8 to 22 days) in which several metestrous or diestrous smears were obtained. However, no consistent differences between the three genotypes were observed. In all groups regular estrous cycles with a length of 4 to 6 days were found (results not shown).

### Weights of ovaries and uterus

Uterine and ovarian weights were determined to obtain an indication whether AMH had some effect on development of the uterus and the ovary. Furthermore, the uterine weight is an estrogen-sensitive parameter and thus may also give an indication for ovarian function.

At all ages no statistical difference was found in uterine weight between the three genotypes (Table 1). No difference in the weight of the ovaries between the three groups was found at 25 days and 13 months of age. At the age of 4 months, however, the weight of the two ovaries was about 1.8-fold higher in the AMH (-/-) females than in AMH (+/+) mice. It is of interest to note that the ovarian weight of AMH (+/-) females fell in between the weights of the ovaries of AMH (+/+) and AMH (-/-) mice (Figure 1).

### Ovarian morphology

(Results are not shown.) The ovaries of all genotypes contained primordial, preantral and small antral follicles, both non-atretic and atretic. In 4- and 13-month-old animals both fresh and old corpora lutea were present, indicating an active estrous cycle. At all ages atretic oocytes were found in the interstitium. In a 25-day-old AMH (-/-) mouse ovary many small follicles are found, whereas a 13-month-old AMH (-/-) mouse ovary contains very few small follicles. At this latter age, the main part of the ovary consists of interstitial tissue and corpora lutea. For ovaries of age-matched AMH (+/+) and AMH (+/-) females similar histology was observed, as reported previously by Behringer et al. 1994.

### Follicle counts

To test the hypothesis that AMH might play a role in follicle maturation or selection the entire follicle population in 25-day-, 4- and 13-month-old AMH (+/+), AMH (+/-) and AMH (-/-) female mice was determined (Figures 2A-C). Note the differences in scale of the y-axis between the different graphs in Figures 2A-C.

In 25-day-old females no significant difference in the number of primordial follicles between AMH (+/+), AMH (+/-) and AMH (-/-) females was detected (Figure 2A). However, compared to the ovaries of AMH (+/+) females of 4 months and 13 months old, the ovaries of age-matched AMH (-/-) females contained a significantly smaller number of primordial follicles (Figures 2B and 2C). In fact, at 13 months of age the number of primordial follicles was reduced in AMH (-/-) females to 38 ± 15, while in the AMH (+/+) females the average number of primordial follicles was 225 ± 52 (Figure 2C).

For the category of non-atretic small follicles the AMH (-/-) females at the age of 25 days showed a marked increase of approximately 1.5-fold compared to the AMH (+/+) mice (Figure 2A). In 4-month-old AMH (-/-) females an approximately 3-fold increase in the number of non-atretic small follicles was found compared to the AMH (+/+) females (Figure 2B). This 3-fold increase in follicle numbers was also found for the atretic small follicles. In contrast, significantly less non-atretic and atretic small follicles were counted in the 13-month-old AMH (-/-) females (Figure 2C). The number of atretic oocytes in 4-month-old AMH (-/-) females was about 4-fold larger than in AMH (+/+) females (Figure 2B). In the three groups of mice at the three different ages an average number of 0.9 ± 0.2 non-atretic large follicles and 0.8 ± 0.2 atretic large follicle were found (results not shown).

In almost all instances, the number of follicles present in AMH (+/-) females fell in between the numbers found in the AMH (+/+) and AMH (-/-) females (Figures 2A-C).

### Number of fresh corpora lutea

To determine whether there are any differences between the three genotypes in the number of ovulations during the last estrous cycle, the number of fresh corpora lutea were counted in the 4- and 13-month-old females. At both 4 and 13 months of age no significant differences between the three groups of mice were found and an average number of 10 fresh corpora lutea was present (results not shown).

### Measurement of serum FSH and inhibin levels

In 4-month-old AMH (-/-) females the serum inhibin level was 2-fold higher than in AMH (+/+) females of the same age, while in 13-month-old AMH (-/-) females the inhibin serum level was 2-fold lower (Table 2). No statistically significant difference was found in inhibin values in 25-day-old females of the three genotypes. For the serum FSH level a statistically significant difference was found only for AMH (-/-) females of 4 months old, which showed a decreased level as compared to AMH (+/+) animals (Table 2).

**Table 1.**

| Uterine weight in AMH (+/+), AMH (+/-) and AMH (-/-) female mice of 25 days, 4 months and 13 months of age. | | |
|---|---|---|
| **Age** | **Genotype** | **Uterus weight (mg)** |
| 25 days (n = 4) | AMH (+/+) | 16.0 ± 3.0 |
| | AMH (+/-) | 17.0 ± 3.0 |
| | AMH (-/-) | 11.0 ± 1.0 |
| 4 months (n = 4) | AMH (+/+) | 130.0 ± 8.0 |
| | AMH (+/-) | 114.9 ± 9.2 |
| | AMH (-/-) | 88.9 ± 7.0 |
| 13 months (n = 5) | AMH (+/+) | 210.5 ± 32.2 |
| | AMH (+/-) | 213.8 ± 18.7 |
| | AMH (-/-) | 165.6 ± 24.5 |
| Values represent the mean ± SEM. | | |

**Table 2.**

| Serum inhibin and FSH levels in AMH (+/+), AMH (+/-) and AMH (-/-) female mice of 25 days, 4 months and 13 months old. | | | |
|---|---|---|---|
| **Age** | **Genotype** | **Inhibin α-subunit (U/ml)** | **FSH (ng/ml)** |
| 25 days (n = 4) | AMH (+/+) | 33.9 ± 5.8 | 24.1 ± 5.3 |
| | AMH (+/-) | 28.9 ± 4.8 | 17.7 ± 1.5 |
| | AMH (-/-) | 25.0 ± 2.2 | 15.0 ± 1.8 |
| 4 months (n = 4) | AMH (+/+) | 20.6 ± 1.6 | 39.7 ± 2.6 |
| | AMH (+/-) | 35.2 ± 1.5 ^{a} | 31.5 ± 2.8 |
| | AMH (-/-) | 43.5 ± 4.0 ^{a} | 27.9 ± 2.9 ^{a} |
| 13 months (n = 5) | AMH (+/+) | 21.5 ± 1.6 | 28.8 ± 2.6 |
| | AMH (+/-) | 12.6 ± 3.8 ^{a} | 31.3 ± 2.9 |
| | AMH (-/-) | 11.5 ± 0.9 ^{a} | 37.9 ± 3.8 |
| Values represent the mean ± SEM. | | | |

| | | | |
|---|---|---|---|
| ^{a} Significantly different from AMH (+/+) in the same age group (P < 0.05), evaluated by Duncan's new multiple range test. | | | |

## Claims

1. A method of treatment of a woman to modify her fertility comprising administering anti-Müllerian hormone (AMH) and/or an AMH agonist and/or an AMH antagonist to said woman in an amount effective to modify fertility.

2. The method of claim 1 wherein AMH and/or an AMH agonist and/or an AMH antagonist are administered to said woman to inhibit recruitment of primordial follicles into the pool of growing follicles.

3. The method of claim 2 wherein AMH and/or an AMH agonist and/or an AMH antagonist are administered to said woman to obtain contraception and/or prolong the duration of fertility and/or postpone the menopause.

4. The method of claim 2 or 3 wherein AMH and/or an AMH agonist is administered.

5. The method of any one of claims 2 to 4 further comprising administration of a different contraceptive.

6. The method of claim 1 wherein AMH and/or an AMH agonist and/or an AMH antagonist are administered to said woman to promote recruitment of primordial follicles into the pool of growing follicles.

7. The method of claim 6 wherein AMH and/or an AMH agonist and/or an AMH antagonist are administered to said woman to treat ovarian failure caused by lack of proper recruitment of primordial follicles into the pool of growing follicles and/or to promote fertility and/or to shorten the duration of the menopause.

8. The method of claim 6 or 7 wherein an AMH antagonist is administered.

9. The method of any one of claims 6 to 8 further comprising administration of a fertility hormone like FSH or LH.

10. Anti-Müllerian hormone (AMH), an AMH agonist or an AMH antagonist for use in human medical therapy.

11. Use of anti-Müllerian hormone (AMH) and/or an AMH agonist and/or an AMH antagonist in the manufacture of a medicament for modifying fertility in women.

12. A pharmaceutical formulation comprising anti-Müllerian hormone (AMH) and/or an AMH agonist and/or an AMH antagonist in combination with a physiologically acceptable diluent or carrier.

13. The pharmaceutical formulation of claim 12 for modifying fertility in women.

14. A method of estimating the remaining duration of the fertile period and/or the onset of the menopause and/or the course of the perimenopausal transition of a woman comprising performing an assay on an appropriate tissue or body fluid sample from said woman, for example a blood, plasma or serum sample, to determine the anti-Müllerian hormone (AMH) level.
